# EUROPÄISCHE PATENTSCHRIFT

(11) **EP 1 692 997 B1**
(45) Veröffentlichungstag und Bekanntmachung des Hinweises auf die Patenterteilung: **08.07.2015**
(21) Anmeldenummer: 06003478.2
(22) Anmeldetag: 21.02.2006
(51) Int. Cl.: A61B 3/12

(54) **Verfahren und Vorrichtung zur Bestimmung einer Durchblutungsstörung**
Method and device for determing a circulatory disorder
Procédé et dispositif pour constater des troubles de la circulation sanguine

(30) Priorität: 21.02.2005 DE 102005008106
(43) Veröffentlichungstag der Anmeldung: 23.08.2006
(73) Patentinhaber: Tornow, Ralf, 91056 Erlangen (DE)
(72) Erfinder: Tornow, Ralf, 91056 Erlangen (DE)
(74) Vertreter: Engel, Christoph Klaus

(56) Entgegenhaltungen:
- EP-A- 0 284 248
- US-A- 3 948 248
- US-A- 5 543 866
- US-B1- 6 347 242
- US-B1- 6 621 917
- SCHMETTERER L ET AL: "Laser interferometric investigations of pulsatile choroidal blood flow: review and new results on the validity of the technique" JOURNAL OF BIOMEDICAL OPTICS SPIE USA, Bd. 3, Nr. 3, Juli 1998 (1998-07), Seiten 246-252, XP002382290 ISSN: 1083-3668

## Beschreibung

Die vorliegende Erfindung betrifft ein Verfahren und eine Vorrichtung zur Bestimmung zeitlich abweichender Durchblutungsänderungen in den Augen, die einen Hinweis auf das Vorliegen einer Durchblutungsstörung, insbesondere von Störungen der Hämodynamik am menschlichen Auge geben können.

Es gibt unterschiedliche Gründe, die okuläre Hämodynamik zu erfassen. Eine gestörte Durchblutung des Auges steht häufig im Zusammenhang mit Augenerkrankungen, wie beispielsweise Glaukom, diabetische Retinopathie und altersbedingte Makulardegeneration, bei denen es sich um häufige Ursachen einer Erblindung handelt. Wenn Durchblutungsstörungen am Auge rechtzeitig erkannt werden, sind die Aussichten einer frühzeitigen Diagnose der ursächlichen Erkrankung und einer erfolgreichen Behandlung groß.

Aus dem Stand der Technik sind unterschiedliche Verfahren zur Bestimmung der Parameter der okulären Hämodynamik bekannt. Beispielsweise kann die Strömungsgeschwindigkeit in den Blutgefäßen des Auges durch Ultraschall oder Laser-Doppler-Geschwindigkeitsmessungen bestimmt werden.

Die US 6,347,242 B1 beschreibt ein Verfahren und ein Gerät zur Messung der Durchblutung der Augen. Die Durchblutung im Auge wird dabei mit Hilfe der Angiografie gemessen, was jedoch die Injektion eines Kontrastmittels erfordert. Dieses Gerät umfasst Glasfaserkabel an deren Enden sich spezielle Kontaktobjektive zum gleichzeitigen Aufsetzen auf die Augen befinden. An den Enden der Glasfaserkabel befinden sich Fotodetektoren. Die Ausgangssignale der Detektoren werden synchron aufgezeichnet und mit einem PC ausgewertet.

Generell wird bei der Angiographie ein Kontrastmittel in eine Vene injiziert. Das zu untersuchenden Gebiet (hier das Auge) wird mit einer Lichtquelle beleuchtet, die den Farbstoff zum Fluoreszieren anregt. Wenn der im Blut gelöste Farbstoff in das Auge einströmt, steigt die Intensität des Fluoreszenzlichtes an, erreicht ein Maximum und nimmt dann wieder ab. Das Einströmen des Farbstoffes zeigt im wesentlichen den konstanten Anteil des Blutflusses an. Die Angiographie hat u.a. den Nachteil, dass der Farbstoff allergische Reaktionen auslösen kann.

Die US 6,086,205 A zeigt eine Gerät und ein Verfahren zur beidseitigen Angiographie der Netzhaut. Das Gerät umfasst zwei optische Module, welche an beiden Augen positioniert werden.

Auch die Messung der durchblutungsbedingten Änderung des Augeninnendrucks oder die durchblutungsbedingte Änderung der Augenlänge können Aufschluss über den aktuellen Durchblutungszustand des Auges geben.

Die US 3,948,248 A betrifft ein Verfahren zur Bestimmung des Okularpulses. Hierfür wird kontinuierlich ein Ultraschallsignal auf das Auge gegeben und das reflektierte Signal ausgewertet. Es werden beide Augen gleichzeitig untersucht und es wird die Differenz der Signale beider Augen gebildet.

Bei vielen bisher bekannten Verfahren erfolgt jeweils die Vermessung des Durchblutungszustandes eines Auges bzw. beider Augen zeitlich nacheinander, woraufhin die gewonnenen Werte mit Normwerten verglichen werden, die auf der Vermessung der Augen von gesunden Personen beruhen. Ein Problem solcher Vergleichsmessungen besteht darin, dass eine natürliche Streuung der Messwerte bei unterschiedlichen Personen auftritt, so dass aus einer nur geringfügigen Abweichung von einem empirisch ermittelten Normwert noch nicht ohne Weiteres auf das Vorliegen einer Durchblutungsstörung des vermessenen Auges geschlussfolgert werden kann. Außerdem sind die Messwerte sehr stark zeitabhängig, da die Durchblutung einer natürlichen Schwankung auf Grund einer veränderten Pulsfrequenz oder des variierenden Blutdrucks unterworfen ist. Anfängliche Durchblutungsstörungen, die zunächst nur ein geringes Ausmaß besitzen, lassen sich mit diesen Verfahren daher nicht zuverlässig ermitteln, jedenfalls nicht mit einem vertretbaren Zeit- und Kostenaufwand.

Die Aufgabe der vorliegenden Erfindung besteht somit darin, ein Verfahren und eine Vorrichtung bereit zu stellen, die es gestatten, mit geringem Aufwand das Vorliegen von Durchblutungsstörungen am Auge durch berührungslose und nicht-invasive Messungen mit erhöhter Sicherheit festzustellen. Dabei soll auch auf die Injektion von Farbstoffen verzichtet werden können, um die Nachteile der Angiographie zu vermeiden. Weiterhin ist es ein Ziel, die gewonnenen Informationen über den Durchblutungszustand des Auges so aufzubereiten, dass dieser die Basis für eine Diagnose über mögliche Augenerkrankungen bilden kann oder zumindest Entscheidungshilfe dafür ist, weitere Untersuchungen einzuleiten.

Gelöst wird diese Aufgabe durch das im Anspruch 1 angegebene Verfahren bzw. durch die im Anspruch 4 näher definierte Vorrichtung.

Die Erfindung beruht auf der Erkenntnis, dass krankheitsbedingte Veränderungen der Durchblutung der Augen üblicherweise zuerst an einem der beiden Augen des Patienten auftreten. Ein direkter Vergleich der zeitabhängigen Durchblutungsänderung an beiden Augen kann somit bereits kleine Veränderungen der okulären Hämodynamik aufzeigen. Eine beginnende Erkrankung auf nur einem Auge hat nämlich zur Folge, dass die Messwerte der zeitlichen, pulsbasierten Durehblutungsänderung bei beiden Augen unterschiedlich sind.

Um verlessliche Aussagen treffen zu können, muss die Durchblutungsänderung jedoch exakt synchron an beiden Augen des Probanden gemessen werden. Relevante Messwerte, die auf die Durchblutung schließen lassen, wie beispielsweise die Blutflussgeschwindigkeit, der Augeninnendruck oder die Reflexion der Netzhaut, unterliegen herzschlagbedingten pulsatilen Schwankungen. Der Vergleich von pulsatilen Signalen, die zu unterschiedlichen Zeiten aufgenommen werden, ist auf Grund der physiologischen Schwankungen im Herx-Kreislauf-System (schwankende Herzrate, schwankendes Schlagvolumen, schwankender Blutdruck usw.) für die Beurteilung der Unterschiede in den Durchblutungsänderungen der beiden Augen nicht aussagekräftig.

Die erfindungsgemäße synchrone Messung der Durchblutungsänderung an beiden Augen hat demgegenüber den Vorteil, dass solche physiologischen Schwankungen keinen Einfluss auf die gewonnenen Messwerte haben. Bei einem allseits intakten Blutgefäßsystem werden die Durchblutungsänderungen in beiden Augen nahezu synchron ablaufen. Pathologisch bedingte kleine Abweichungen der Änderung der Durchblutung in einem Auge lassen sich auf diese Weise durch den direkten Vergleich mit dem gesunden Auge leicht feststellen.

Ein besonderer Vorteil des erfindungsgemäßen Verfahrens ist, dass es sich um eine echte nicht-invasive Methode handelt. Im Gegensatz zur Angiografie müssen dem Probanden keine Kontrastmittel injiziert werden. Die Augen können ohne eine serührung untersucht werden, was für den Probanden wesentlich angenehmer ist und Desinfektionen oder Hornhautverletzungen ausschließt.

Es ist jedoch darauf hinzuweisen, dass allein die Feststellung von Unterschieden zwischen den zeitabhängigen Durchblutungsänderungen der beiden Augen noch keine abschließende Diagnose zur Art einer vorliegenden Erkrankung darstellt. Unterschiedliche Durchblutungsänderungen sind zunächst als Hinweis darauf zu verstehen, dass Unterschiede in den Gefäßsystemen bestehen, die das jeweilige Auge mit Blut versorgen. Eine Schädigung kann beispielsweise in den unmittelbar an die Netzhaut angrenzenden Gefäßen oder auch in der jeweiligen Halsschlagader vorliegen. Die Bewertung der mit dem erfindungsgemäßen verfahren gewonnenen Messergebnisse bleibt somit dem Mediziner vorbehalten und wird vom Schutzbereich dieses Patents nicht tangiert.

Gemäß einer bevorzugten Ausführungsform des erfindungsgemäßen. Verfahrens wird die zeitliche Durchblutungsänderung durch eine optische Messung der relativen Blutdurehflussmenge im Bereich der Netzhaut, insbesondere im Bereich der Papille (blinder Fleck) bestimmt. Dazu wird die Papille bevorzugt mit grünem Licht bestrahlt, welches vom Blut besonders gut absorbiert wird. Der Grad der verbleibenden Lichtreflexion ist umgekehrt proportional zu der im jeweiligen Zeitpunkt den bestrahlten Bereich durchströmenden Blutmenge. Eine starke Lichtreflexion bedeutet ein geringes Blutvolumen, während bei einer niedrigen Reflexion ein hohes Blutvolumen vorhanden ist, so dass die Lichtabsorption hoch ist. Durch eine gerichtete Bestrahlung der Papille ist die Lichtbelastung der Augen insgesamt niedrig.

Bei abgewandelten Ausführungsformen kann die zeitabhängige Durchblutungsänderung aber auch mit anderen Messverfahren bestimmt werden. Prinzipiell sind solche Messverfahren aus dem Stand der Technik bekannt, so dass auf eine detaillierte Erläuterung an dieser Stelle verzichtet werden kann. Der Blutdurchfluss kann beispielsweise mit Laser-Doppler- oder Ultraschallverfahren bestimmt werden. Auch der sich ändernde Augeninnendruck ist ein Maß für die Durchblutungsänderung. Ebenso ist es bekannt, dass sich die Augenlänge in Abhängigkeit von der pulsatilen Durchblutung ändert, so dass beispielsweise eine interferometrisch bestimmte Längenänderung des Augapfels als Maß für die Durchblutungsänderung genutzt werden kann.

Gemäß einer bevorzugten Ausführungsform erfolgt die Auswertung der Unterschiede der Durchblutungsänderung an beiden Augen durch eine grafische Darstellung der Messwerte. Geringfügige Abweichungen lassen sich besser sichtbar machen, wenn die Messwerte des einen Auges als Funktion der Messwerte des anderen Auges in einem Diagramm dargestellt werden. Bei abgewandelten Ausführungsformen können auch Korrelationsparameter bestimmt werden, um die Abweichungen ersichtlich zu machen.

Bestimmte Durchblutungsstörungen führen zu einer verstärkten Differenz der zeitabhängigen Durchblutungsänderung an beiden Augen, wenn vor und/oder während der Messung der Durchblutungsänderung eine Anregung der Durchblutung durch Bestrahlung der Netzhaut mit einem Flickerlicht erfolgt.

Eine kontinuierliche Messung unter Flickerliehtbeleuchtung ist möglich, da das erfindungsgemäße Verfahren im Gegensatz z.B. zur Angiografie sehr oft ohne Einschränkungen wiederholt werden kann. Die Flickerlichtprovokation ermöglicht erstmals eine Differenzierung von Durchblutungsstörungen an der Halsschlagader oder am Auge, sodass Augenerkrankungen klar von Pathologien der Halsschlagader abgegrenzt werden können. Zudem können Unterschiede in der Dynamik der Reaktion beider Augen auf die Flickerlichtprovokation gemessen werden.

Darüber hinaus können zur Verbesserung des Messergebnisses auch andere Maßnahmen ergriffen werden, die sich auf die Durchblutung des Auges, insbesondere der Netzhaut auswirken.

Ein wesentliches Merkmal der durch die Erfindung angegebenen Vorrichtung zur Bestimmung einer Durchblutungsstörung besteht darin, dass Mittel vorhanden sind, die eine synchrone Bestimmung der zeitabhängigen Durchblutungsänderung in beiden Augen des Probanden gestatten. Um die Durchblutungsänderung in beiden Augen zu messen, können dabei verschiedene Messmittel zum Einsatz kommen. Bevorzugt wird wiederum die Erfassung einer zeitabhängigen Reflexion von Licht, welches auf die Netzhaut projiziert wurde und teilweise von dem durchströmenden Blut absorbiert wird. Das reflektierte Licht kann durch Bildaufnahmegeräte oder auch durch einfache optische Sensoren erfasst werden.

Besonders vorteilhaft ist der Einsatz einer sogenannten Funduskamera (erhältlich von der Fa. Carl Zeiss, Typ FF450), welche die relative Fundusreflektion und damit den Durchblutungszustand im Bereich der Papille aufzeichnet. Dabei ist es erforderlich, dass entweder zwei Funduskameras vor den Augen des Probanden platziert werden oder dass entsprechende Optiken zum Einsatz kommen, die eine gleichzeitige Aufnahme der Netzhaut beider Augen mit einer einzigen Kamera gestatten. Die Aufnahme der Fundus mit Kameras ermöglicht die Kombination der hohen Ortsauflösung einer abbildenden Methode mit der hohen Zeitauflösung einer Methode, welche die herzschlagsynchrone Pulsation ausnutzt. Dadurch wird die Auswertung insbesondere des Anstieges der Pulskurve sehr präzise und lässt selbst kleine Änderungen erkennen, die mit den Geräten und Verfahren nach dem Stand der Technik nicht ermittelbar gewesen sind.

Die Unterschiede zwischen den zeitabhängigen Durchblutungsänderungen können in unterschiedlichen Amplituden der Messsignale festgestellt werden. Voneinander abweichende Durchblutungsänderungen der beiden Augen können sich aber auch in unterschiedlichen Anstiegszeiten bzw. zeitlichen Verzögerungen bei der Änderung der Durchblutung bemerkbar machen.

Die erfindungsgemäße Vorrichtung weist vorzugsweise Datenverarbeitungsmittel auf, mit denen solche Unterschiede rechentechnisch festgestellt werden können. Die von den Bildaufzeiehnungselementen oder optischen Sensoren gelieferten Messdaten werden dazu miteinander verglichen. Obwohl die heutzutage zur Verfügung stehende Rechentechnik zu einer synchronen Datenverarbeitung in der Lage ist, können die Messwerte zunächst in einem Speicher abgelegt werden und nachfolgend entsprechenden Vergleichsroutinen unterzogen werden. Ebenso können herkömmliche Messdatenfilter und Anzeigeelemente eingesetzt werden, um bestehende Unterschiede bei der Durchblutungsänderung komfortabel darzustellen.

Weitere Vorteile, Einzelheiten und Weiterbildungen der vorliegenden Erfindung ergeben sich aus der nachfolgenden Beschreibung bevorzugter Ausführungsformen unter Bezugnahme auf die Zeichnung. Es zeigen:
- Fig. 1: eine schematische Darstellung einer Vorrichtung zur Bestimmung einer Durchblutungsstörung;
- Fig. 2: Diagrammdarstellungen typischer Messsignale von zeitabhängigen Durchblutungsänderungen und der bestimmten Unterschiede der Durchblutungsänderungen im linken und im rechten Auge.

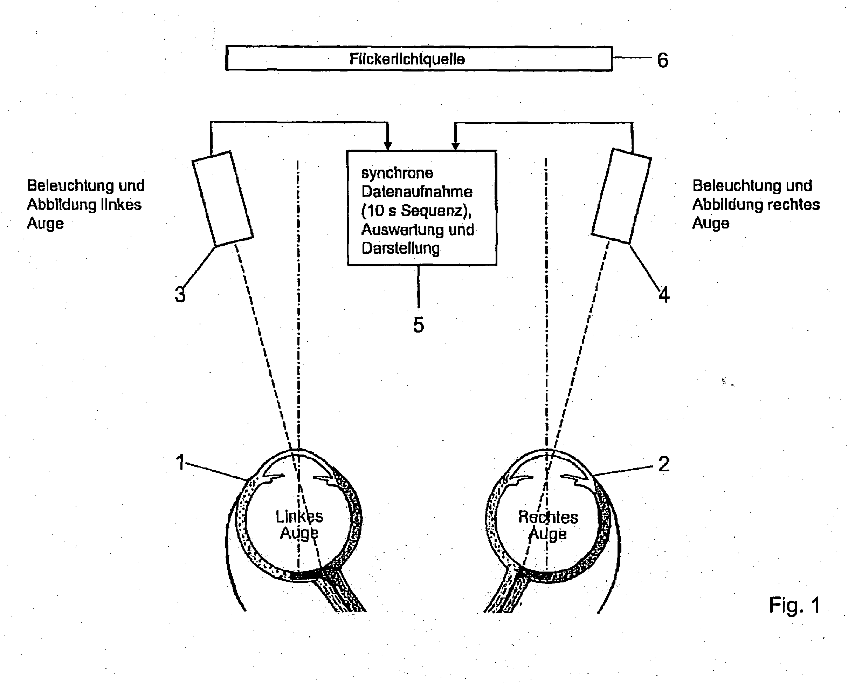

In Fig. 1 ist eine prinzipielle Darstellung einer Vorrichtung gezeigt, mit welcher über die Erfassung zeitabhängiger Durchblutungsänderungen in beiden Augen eines Probanden eine Durchblutungsstörung bestimmt werden kann. Dafür ist eine synchrone Bestimmung der zeitabhängigen Durchblutungsänderungen in beiden Augen erforderlich. Das linke Auge 1 und das rechte Auge 2 eines Probanden werden bei der dargestellten Ausführungsform gleichzeitig beleuchtet und die Reflexionen von der Netzhaut des jeweiligen Auges werden aufgezeichnet. Dazu dienen eine linke Bilderfassungseinheit 3 und eine rechte Bilderfassungseinheit 4.

Die Bilderfassungseinheiten 3, 4 projizieren vorzugsweise grünes Licht mit einer Wellenlänge von 548nm ± 10nm auf die Papille des jeweils zugeordneten Auges, da dies der Bereich der höchsten Durchblutung der Netzhaut ist. Gleichzeitig wird das vom beleuchteten Netzhautbereich reflektierte Licht von den Bilderfassungseinheiten 3, 4 aufgezeichnet. Vorzugsweise wird dazu jeweils eine ZEISS Funduskamera eingesetzt. Die Messung der Lichtreflexion kann aber auch über optische Sensoren erfolgen.

Bei abgewandelten Ausführungsformen kann der Proband die erforderlichen Einstellungen wie z.B. den Augenabstand, den Dioptrienausgleich und die Fokussierung wie bei einem Fernglas selbstständig vornehmen. Die genannten Funduskameras können auch in ein tragbares, batteriebetriebenes Gerät integriert sein, um mobile Messungen beispielsweise bei Reihenuntersuchungen zu ermöglichen.

Über einen vorgegebenen Zeitraum, der mehrere Herzschläge umfassen sollte, werden die von den Bilderfassungseinheiten 3, 4 aufgenommenen Lichtreflexion synchron als Messwerte aufgezeichnet. Dazu dient beispielsweise eine Datenverarbeitungseinheit 5. Die aufgezeichneten Datensequenzen, die z.B. jeweils eine Dauer von 10 s umfassen, werden in der Datenverarbeitungseinheit ausgewertet und zur Anzeige gebracht, wobei insbesondere die Unterschiede der Durchblutungsänderungen der beiden Augen von Interesse sind.

Zur Anregung der Durchblutung der Netzhaut und damit zur Verbesserung des Messergebnisses kann eine Flickerlichtquelle 6 vorgesehen sein, die an beide Augen ein Flickerlicht mit einer Frequenz von beispielsweise 15 Hz bereitstellt. Die Flickerlichtquelle lässt sich unmittelbar in die Messvorrichtung integrieren oder als eigenständige Einheit gestalten.

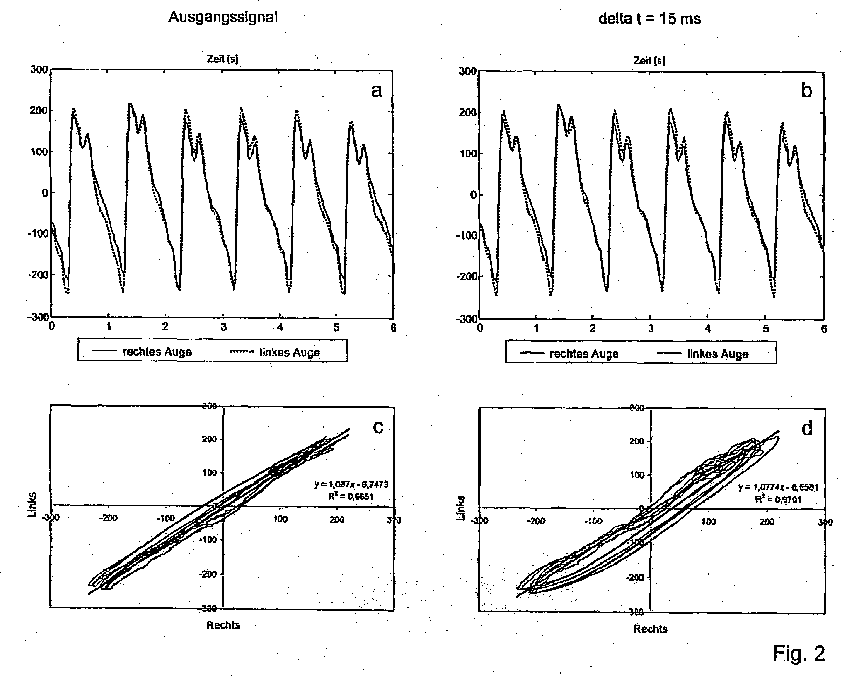

Fig. 2 zeigt vier Diagramme zur Verdeutlichung der mit der zuvor beschriebenen Vorrichtung erzielbaren Messdaten und deren Verarbeitungs- und Darstellungsmöglichkeiten.

Im Diagramm a) ist der typische Signalverlauf der gemessenen Durchblutungsänderungen am linken und am rechten Auge dargestellt. Es ist ersichtlich, dass die Durchblutungsänderung über die Zeit dem Pulsschlag des Probanden folgt. Die an den beiden Augen aufgenommenen Messdaten verlaufen im Wesentlichen parallel zueinander, wobei die Ursachen der teilweisen Abweichungen der Kurvenverlaufe zunächst unklar bleiben - aber auch nicht von Interesse sind.

Im Diagramm b) ist ein typischer Signalverlauf einer veränderten Durchblutungsänderung dargestellt, der sich von der Datenaufzeichnung gemäß Diagramm a) dahingehend unterscheidet, dass ein zeitlicher Versatz zwischen den die Durchblutungsänderung der beiden Augen repräsentierenden Kurvenverläufen besteht. Im dargestellten Beispiel beträgt dieser Zeitversatz Δt = 15 ms. Ein solcher zeitlicher Versatz kann beispielsweise entstehen, wenn auf Grund einer Gefäßverengung die vom Herzschlag vorgegebene Durchblutungsänderung an einem Auge verzögert wirksam wird. In der Praxis wird eine derartige Durchblutungsstörung voraussichtlich nicht nur eine zeitliche Verzögerung, sondern auch eine Amplitudenveränderung mit sich bringen. Der Einfachheit halber wird hier jedoch nur die durch die Durchblutungsstörung ausgelöste zeitliche Verzögerung betrachtet.

Ein bildlicher vergleich der Kurvenverläufe in den Diagrammen a) und b) lässt einen Unterschied der pulsativen Durchblutungsänderung an den beiden vermessenen Augen kaum erkennen. Noch viel weniger würde eine mögliche Durchblutungsstörung bei einem Vergleich der aufgenommenen Messwerte mit Normwerten feststellbar sein, wie dies bislang im Stand der Technik bei einer Einzelaugenuntersuchung erforderlich wäre.

Um eine mögliche krankhafte Durchblutungsstörung erkennen zu können, müssen die Unterschiede der Durchblutungsänderungen besser hervorgehoben werden. Dies ist beispielsweise mit einer grafischen Darstellung möglich, bei der die am rechten Auge aufgenommenen Messwerte über den Messwerten des linken Auges zum jeweiligen Zeitpunkt aufgetragen werden (Rechts-Links-Darstellung). Das Diagramm c) zeigt das Ergebnis dieser Darstellungsart unter Zugrundelegung der Messreihen, die im Diagramm a) dargestellt wurden. Da in diesem Fall die Durchblutungsänderungen an beiden Augen nahezu synchron verlaufen, ergibt sich im Diagramm c) eine Kurvendarstellung, die an eine Gerade angenähert ist und im Idealfall exakt einer Gerade entsprechen würde, die bei gleicher Achsenkalibrierung in einem Winkel von 45° in der Diagrammfläche verläuft.

Die Rechts-Links-Darstellung im Phasenraum lässt die Ergebnisse auf einen Blick erkennen, auch wenn mehrere Pulssehläge gleichzeitig dargestellt sind. Zudem können in einer solchen Darstellung Unterschiede in der Dynamik der Reaktion beider Augen auf die Flickerlichtprovokation leicht erkannt werden.

Das Diagramm d) zeigt die erwähnte Rechts-Links-Darstellung der an den beiden Augen aufgenommenen Messkurven, wie sie im Diagramm b) über der Zeit dargestellt wurden. Es ist ersichtlich, dass es auf Grund des Zeitversatzes zwischen den Messdatenreihen zu einer elliptischen Aufweitung der Kurve kommt. Aus der Darstellungsart in Diagramm d) ist daher sofort ersichtlich, dass die Durchblutungsänderungen in den beiden Augen nicht synchron verlaufen. Sofern ein entsprechend konfiguriertes Gerät eine solche Anzeige ausgibt, ist der fehlende Gleichlauf der Durchblutungsänderung leicht erkennbar, so dass ein das Gerät benutzender Mediziner Veranlassung haben wird, weitere Untersuchungen vorzunehmen, um die Ursache der ungleichmäßigen Durchblutungsänderungen festzustellen, die in einer krankhaften Durchblutungsstörung liegen kann.

Die oben erwähnte Datenverarbeitungseinheit wird vorzugsweise geeignete Messdatenfilter enthalten oder durch entsprechende Softwareroutinen nachbilden, um Messfehler aus den ermittelten Daten der Durchblutungsänderung auszufiltern. Solche Fehler können beispielsweise aus Augenbewegungen während der Messung resultieren. Ebenso würden sich Intensitätsschwankungen der Projektionslichtquellen verfälschend auf das Messergebnis auswirken, so dass derartige Einflüsse vor der weiteren Datenverarbeitung zu filtern sind.

Ergänzend wird darauf hingewiesen, dass die Bestimmung einer Durchblutungsstörung durch die synchrone Messung gleichlaufender Durchblutungsänderungen auch an anderen Körperpositionen möglich ist, soweit zwei im Wesentlichen spiegelbildlich ausgebildete Gefäßsysteme überprüft werden können. Beispielsweise können Durchblutungsänderungen, die dem Herzrhythmus folgen durch Impedanzmessungen an den Halsschlagadern überwacht und miteinander verglichen werden.

### Bezugszeichenliste

- 1 -: linkes Auge
- 2 -: rechtes Auge
- 3 -: linke Bilderfassungseinheit
- 4 -: rechte Bilderfassungseinheit
- 5 -: Datenverarbeitungseinheit
- 6 -: Flickerlichtquelle

## Patentansprüche

1. Verfahren zur Bestimmung einer Durchblutungsstörung, bei welchem die zeitabhängige Durchblutungsänderung synchron an beiden Augen (1; 2) des Probanden über einen vorgegebenen Zeitraum gemessen wird, wobei ein Vergleich zwischen Messwerten erfolgt, die über mehrere Herzschläge hinweg aufgenommen werden, um Unterschiede zwischen der zeitabhängigen Durchblutungsänderung des linken und des rechten Auges (1; 2) zu bestimmen, **dadurch gekennzeichnet, dass** die Durchblutungsänderung durch eine optische Messung der relativen Blutdurchflussmenge im Bereich der Netzhaut im Bereich der Papille bestimmt wird, wobei der Vergleich der am linken und rechten Auge (1; 2) aufgenommenen Messwerte:
• durch Darstellung der Messwerte in einem Diagramm erfolgt, wobei die Messwerte des einen Auges (2) als Funktion der Messwerte des anderen Auges (1) aufgetragen werden; oder
• durch Bestimmung der Korrelation zwischen diesen Messwerten erfolgt; oder
• anhand von sich unterscheidenden Anstiegszeiten oder von zeitlichen Verzögerungen bei der Änderung der Durchblutung erfolgt.

2. Verfahren nach Anspruch 1, **dadurch gekennzeichnet, dass** Licht mit einer Wellenlänge, die vom Blut gut absorbiert wird, auf die Netzhaut projiziert und das reflektierte Licht gemessen wird.

3. Verfahren nach Anspruch 1 oder 2, **dadurch gekennzeichnet, dass** vor und/oder während der Messung der Durchblutungsänderüng eine Anregung der Durchblutung durch Bestrahlung der Netzhaut mit einem Flickerlicht erfolgt.

4. Vorrichtung zur Bestimmung einer Durchblutungsstörung bei einem Probanden, umfassend:
- Mittel (3, 4) zur synchronen Bestimmung der zeitabhängigen pulsativen Durchblutungsänderung in beiden Augen (1; 2) des Probanden;
- Mittel (5), welches zur Bestimmung der Unterschiede zwischen Messwerten der zeitabhängigen pulsativen Durchblutungsänderung über die Dauer mehrere Herzschläge im linken und im rechten Auge (1; 2) konfiguriert ist; und
- Mittel (5) zur Darstellung der bestimmten Unterschiede der zeitabhängigen pulsativen Durchblutungsänderung im linken und im rechten Auge (1; 2);
**dadurch gekennzeichnet, dass** die Mittel (3, 4) zur synchronen Bestimmung der zeitabhängigen pulsativen Durchblutungsänderüng in beiden Augen (1; 2) mindestens eine Lichtquelle (6) zur Projektion von Licht auf die Netzhaut beider Augen (1; 2) und Messmittel (3; 4) zur Erfassung der zeitabhängigen Reflexion des Lichts von der Netzhaut beider Augen (1; 2) umfassen; wobei das Mittel (5) zur Bestimmung der Unterschiede zwischen der zeitabhängigen Durchblutungsänderung im linken und im rechten Auge (1; 2) zu vorbestimmten Zeitpunkten einen Vergleich der von den Mitteln (3, 4) zur synchronen Bestimmung der zeitabhängigen Durchblutungsänderung gelieferten Messdaten ausführt; und wobei der Vergleich der am linken und rechten Auge (1; 2) aufgenommenen Messdaten:
• durch Darstellung der Messdaten in einem Diagramm erfolgt, wobei die Messdaten des einen Auges (2) als Funktion der Messdaten des anderen Auges (1) aufgetragen werden; oder
• durch Bestimmung der Korrelation zwischen diesen Messwerten erfolgt; oder
• für die Amplitude, die Anstiegszeit oder die zeitliche Verzögerung der von den Mitteln (3, 4) zur synchronen Bestimmung der zeitabhängigen Durchblutungsänderung gelieferten Messdaten erfolgt.

5. Vorrichtung nach Anspruch 4, **dadurch gekennzeichnet, dass** die Lichtquelle (6) grünes Licht, vorzugsweise mit einer Wellenlänge von 548 nm ± 10 nm emittiert.

6. Vorrichtung nach Anspruch 4 oder 5, **dadurch gekennzeichnet, dass** die Messmittel zur Erfassung der zeitabhängigen Reflexion des Lichts aus der folgenden Gruppe ausgewählt sind:
- Bildaufnahmegeräte, insbesondere Funduskameras (3; 4);
- optische Sensoren.

7. Vorrichtung nach einem der Ansprüche 4 bis 6, **dadurch gekennzeichnet, dass** die Mittel zur Bestimmung der Unterschiede zwischen der zeitabhängigen Durchblutungsänderung im linken und im rechten Auge durch eine Datenverarbeitungseinheit (5), insbesondere einen speziell programmierten Computer gebildet sind und dass die Mittel (5) zur Darstellung der bestimmten Unterschiede der zeitabhängigen Durchblutungsänderung eine graphische Anzeige umfassen, auf welcher diese Unterschiede als Kurvenverlauf dargestellt werden.

8. Vorrichtung nach einem der Ansprüche 4 bis 7, **dadurch gekennzeichnet, dass** sie weiterhin eine Flickerlichtquelle (6) umfasst, die vor und/oder während der synchronen Bestimmung der zeitabhängigen Durchblutungsänderung ein Flickerlicht auf beide Augen (1; 2) des Probanden projiziert.

## Claims

1. A method for determining a circulation disorder, in which the time-dependent change in circulation is measured synchronously on both eyes (1; 2) of the proband over a predefined period of time, wherein a comparison is made between measured values recorded over several heartbeats in order to determine differences between the time-dependent change in circulation of the left and right eyes (1; 2), **characterized in that** the change in circulation is determined by an optical measurement of the relative blood flow in the area of the retina in the area of the optic disk, wherein the comparison of the measured values recorded on the left and right eyes (1; 2) :
• is made by representing the measured values in a diagram wherein the values measured on one eye (2) are plotted as a function of the values measured on the other eye (1); or
• is made by determining the correlation between these measured values; or
• is made on the basis of different rise times or delays in the change in circulation over time.

2. The method according to claim 1, **characterized in that** light of a wavelength that is absorbed well by blood is projected onto the retina and the reflected light is measured.

3. The method according to claim 1 or 2, **characterized in that** the circulation is stimulated by irradiation of the retina with a flickering light before and/or during the measurement of the change in circulation.

4. A device for determining a circulation disorder at a proband, comprising:
- means (3, 4) for synchronous determination of the time-dependent pulsatile change in circulation in both eyes (1; 2) of the proband;
- means (5) configured for determining the differences between values measured for the time-dependent pulsatile change in circulation in the left and right eyes (1; 2) over the duration of several heartbeats; and
- means (5) for representing the specific differences in the time-dependent pulsatile change in circulation in the left and right eyes (1; 2);
**characterized in that** the means (3, 4) for synchronous determination of the time-dependent pulsatile change in circulation in both eyes (1; 2) comprise at least one light source (6) for projecting light onto the retina of both eyes (1; 2) and measurement means (3; 4) for detecting the time-dependent reflection of the light by the retina of both eyes (1; 2); wherein the means (5) for determining the differences between the time-dependent change in circulation in the left and right eyes (1; 2) perform a comparison of the measured data supplied by the means (3, 4) for synchronous determination of the time-dependent change in circulation, and wherein the comparison of the measured data recorded on the left and right eyes (1; 2):
• is made by representing the measured data in a diagram, wherein the measured data on the one eye (2) is plotted as a function of the measured data on the other eye (1); or
• by determining the correlation between these measured values; or
• for the amplitude, the rise time or the delay in time of the measurement data supplied by the means (3, 4) for synchronous determination of the time-dependent change in circulation is determined in this way.

5. The device according to claim 4, **characterized in that** the light source (6) emits green light, preferably with a wavelength of 548 nm ± 10 nm.

6. The device according to claim 4 or 5, **characterized in that** the measurement means for detecting the time-dependent reflection of the light are selected from the following group:
- image recording devices, in particular fundus cameras (3; 4);
- optical sensors.

7. The device according to any one of claims 4 to 6, **characterized in that** the means for determining the differences between the time-dependent change in circulation in the left and right eyes are formed by a data processing unit (5), in particular a specially programmed computer and the means (5) for representing the certain differences in the time-dependent change in circulation comprise a graphic display, on which these differences are represented as the shape of a curve.

8. The device according to any one of claims 4 to 7, **characterized in that** it additionally comprises a flickering light source (6), which projects a flickering light onto both eyes (1; 2) of the proband before and/or during the synchronous determination of the time-dependent change in circulation.

## Revendications

1. Procédé destiné à la détermination d'un trouble de la circulation sanguine, dans lequel la modification de la circulation sanguine fonction du temps est mesurée de façon synchrone sur les deux yeux (1 ; 2) du participant à l'examen sur une période prédéfinie, sachant qu'une comparaison entre des valeurs de mesure est effectuée, lesquelles sont enregistrées sur plusieurs pulsations cardiaques, pour déterminer des différences entre la modification de la circulation sanguine fonction du temps de l'oeil gauche et de l'oeil droit (1 ; 2), **caractérisé en ce que** la modification de la circulation sanguine est déterminée par une mesure optique de la quantité relative de débit sanguin au niveau de la rétine au niveau de la papille, sachant que la comparaison des valeurs de mesures enregistrées sur l'oeil gauche et l'oeil droit (1 ; 2) :
• est effectuée par représentation des valeurs de mesure dans un diagramme, sachant que les valeurs de mesure d'un oeil (2) sont rapportées en tant que fonction des valeurs de mesure de l'autre oeil (1) ; ou
• est effectuée par détermination de la corrélation entre ces valeurs de mesure ; ou
• est effectuée à l'aide de temps de montée se différenciant ou de retards temporels lors de la modification de la circulation sanguine.

2. Procédé selon la revendication 1, **caractérisé en ce que** de la lumière ayant une longueur d'onde qui est bien absorbée par le sang est projetée sur la rétine et la lumière réfléchie est mesurée.

3. Procédé selon la revendication 1 ou 2, **caractérisé en ce qu'**avant et/ou pendant la mesure de la modification de la circulation sanguine, la circulation sanguine est excitée par le rayonnement de la rétine avec une lumière scintillante.

4. Dispositif destiné à la détermination d'un trouble de la circulation sanguine chez un participant à l'examen, comprenant :
- des moyens (3, 4) pour déterminer de façon synchrone la modification de la circulation sanguine pulsative fonction du temps dans les deux yeux (1 ; 2) du participant à l'examen ;
- un moyen (5) qui est configuré pour déterminer les différences entre des valeurs de mesure de la modification de la circulation sanguine pulsative fonction du temps sur la durée de plusieurs pulsations cardiaques dans l'oeil gauche et dans l'oeil droit (1 ; 2) ; et
- un moyen (5) pour représenter les différences déterminées de la modification de la circulation sanguine pulsative fonction du temps dans l'oeil gauche et dans l'oeil droit (1 ; 2) ;
**caractérisé en ce que** les moyens (3, 4) de détermination synchrone de modification de la circulation sanguine pulsative fonction du temps dans les deux yeux (1 ; 2) comprennent au moins une source lumineuse (6) pour projeter de la lumière sur la rétine des deux yeux (1 ; 2) et des moyens de mesure (3 ; 4) pour déterminer la réflexion fonction du temps de la lumière de la rétine des deux yeux (1 ; 2) ; sachant que le moyen (5) pour déterminer les différences entre la modification de la circulation sanguine fonction du temps dans l'oeil gauche et dans l'oeil droit (1 ; 2) effectue à intervalles prédéfinis une comparaison des données de mesures fournies par les moyens (3, 4) de détermination synchrone de modification de la circulation sanguine fonction du temps, et sachant que la comparaison des données de mesure enregistrées à l'oeil gauche et à l'oeil droit (1 ; 2) :
• est effectuée par représentation des valeurs de mesure dans un diagramme, sachant que les valeurs de mesure d'un oeil (2) sont rapportées en tant que fonction des valeurs de mesure de l'autre oeil (1), ou
• est effectuée par détermination de la corrélation entre ces valeurs de mesure ; ou
• est effectuée pour l'amplitude, le temps de montée ou le retard temporel des données de mesure fournies par les moyens (3, 4) de détermination synchrone de la modification fonction du temps de la circulation sanguine.

5. Dispositif selon la revendication 4, **caractérisé en ce que** la source lumineuse (6) est de la lumière verte, en particulier avec une longueur d'onde de 548 nm ± 10 nm.

6. Dispositif selon la revendication 4 ou 5, **caractérisé en ce que** les moyens de mesure pour détecter la réflexion fonction du temps de la lumière sont choisis dans le groupe suivait :
- appareils d'enregistrements d'images, en particulier caméras de fond (3 ; 4) ;
- capteurs optiques.

7. Dispositif selon l'une des revendications 4 à 6, **caractérisé en ce que** les moyens de détermination des différences entre la modification de la circulation sanguine fonction du temps dans l'oeil gauche et dans l'oeil droit sont formés par une unité de traitement de données (5), en particulier un ordinateur spécialement programmé, et que les moyens (5) de représentation des différences déterminées de la modification de la circulation sanguine fonction du temps comprennent un affichage graphique sur lequel ces différences sont affichées en tant que tracés de courbes.

8. Dispositif selon l'une des revendications 4 à 7, **caractérisé en ce qu'**il comprend en outre une source de lumière scintillante (6) qui projette une lumière scintillante sur les deux yeux (1 ; 2) du participant à l'examen avant et/ou pendant la détermination synchrone de la modification de la circulation sanguine fonction du temps.
